# EUROPÄISCHE PATENTSCHRIFT

(11) **EP 1 253 964 B1**
(45) Veröffentlichungstag und Bekanntmachung des Hinweises auf die Patenterteilung: **30.08.2006**
(21) Anmeldenummer: 02712803.2
(22) Anmeldetag: 05.01.2002
(51) Int. Cl.: A61M 39/12

(54) **SPANNADAPTER FÜR EINEN KATHETER**
TENSION ADAPTER FOR A CATHETER
ADAPTATEUR DE CONTRAINTE POUR CATHETER

(30) Priorität: 11.01.2001 DE 10100975
(43) Veröffentlichungstag der Anmeldung: 06.11.2002
(73) Patentinhaber: Pajunk, Heinrich, 78187 Geisingen (DE); Pajunk, Horst, 78187 Geisingen (DE)
(72) Erfinder: Pajunk, Heinrich, 78187 Geisingen (DE); Pajunk, Horst, 78187 Geisingen (DE)
(74) Vertreter: Patentanwälte Westphal, Mussgnug & Partner
(86) Internationale Anmeldenummer: PCT/EP2002/000048
(87) Internationale Veröffentlichungsnummer: WO 2002/062417

(56) Entgegenhaltungen:
- EP-A- 0 482 196
- EP-A- 0 596 344
- DE-A- 2 914 034
- GB-A- 2 331 339

## Beschreibung

Die Erfindung betrifft einen Spannadapter für einen Katheter gemäß dem Oberbegriff des Anspruchs 1.

In der Anästhesie werden für länger dauernde operative Eingriffe, für die postoperative Schmerztherapie und für die Behandlung chronischer Schmerzzustände die einen gewissen Körperbereich versorgenden Nerven durch Einleiten eines Anästhetikums blockiert. Zum Einleiten des Anästhetikums oder auch einer sonstigen Flüssigkeit wird ein Katheter benutzt, dessen distales Ende möglichst nahe an dem Nerv positioniert wird, um mit einer möglichst geringen Menge des Anästhetikums eine optimale Wirkung zu erzielen. Damit der Katheter in der gewünschten Lage plaziert werden kann und im Bedarfsfalle auch über eine längere Zeit verbleiben kann, besteht der Katheter aus einem langen dünnen flexiblen Kunststoffschlauch.

Um den flexiblen Katheter in die Nervenscheide oder den Nervenkanal einführen zu können, wird eine Kanüle verwendet, die in die Nervenscheide bzw. den Nervenkanal eingesetzt wird und durch die der Katheter eingeführt wird. Um über den Katheter eine Flüssigkeit z.B. ein Anästhetikum zu applizieren, wird an das extrakorporal verbleibende proximale Ende des Katheters eine Spritze angesetzt. Hierzu dient ein an dem proximalen Ende des Katheters angebrachter Adapter. Soll der Katheter über einen längeren Zeitraum in seiner Lage verbleiben, so muss die zum Einführen des Katheters dienende Kanüle abgezogen werden. Hierzu ist es notwendig, den Adapter von dem Katheter zu entfernen, damit die Kanüle über das proximale Ende des Katheters abgezogen werden kann. Um über den liegenden Katheter das Anästhetikum nachdosieren zu können, muss der Adapter nach dem Abziehen der Kanüle wieder an dem Ende des Katheters angebracht werden. Um den Adapter in einfacher Weise am Ende des Katheters lösbar anzubringen, ist es bekannt, einen Spannadapter der Eingangs genannten Gattung zu verwenden. Dieser Spannadapter enthält ein hohlzylindrisches Spannteil aus einem weichelastischen Material. Das proximale Ende des Katheters wird koaxial in dieses Spannteile eingeführt. Daraufhin wird das Spannteil axial gestaucht, wodurch sich sein lnnendurchmesser verringert und der Katheter festgelegt und an seinem Aussenumfang abgedichtetwird. Ein solcher Spannadapter ist z.B. aus der Patentschrift GB-A-2 331 339 bekannt.

Um das distale Ende des Katheters möglichst genau zu plazieren, ist es bekannt, den Katheter für eine Elektrostimulation des Nerven auszubilden. Hierzu wird in dem Katheter ein dünner Draht angeordnet, der an der distalen Spitze des Katheters mit einer blanken Kontaktspitze den Nerven kontaktiert. Am proximalen Ende des Katheters wird der Draht aus dem Katheter herausgeführt und an einen Stimulator angeschlossen. Bei der Verwendung des bekannten Spannadapters muss der Draht aussen an dem Katheter zurückgeführt werden bis vor das distale Ende des Spannadapters. Dort wird das blankliegende Ende des Drahtes mittels einer Klemme erfasst und kontaktiert. Das Ergreifen des Drahtes mittels der mit dem Stimulator verbundenen Klemme ist mühsam. Zudem sind der Halt der Klemme an dem Draht und die Kontaktierung des Drahtes durch die Klemme unzuverlässig.

Der Erfindung liegt die Aufgabe zugrunde, einen Spannadapter zur Verfügung zu stellen, der ein einfaches und zuverlässiges Kontaktieren eines für die Elektrostimulation ausgebildeten Katheters ermöglicht.

Diese Aufgabe wird erfindungsgemäß gelöst durch einen Spannadapter mit den Merkmalen des Anspruchs 1.

Vorteilhafte Ausführungen der Erfindung sind in den Unteransprüchen angegeben.

Erfindungsgemäß ist in dem Spannadapter eine Kontaktbuchse angeordnet, die sich proximal an das Spannteil anschließt und die mit einem nach aussen geführten elektrischen Anschluß für den Stimulator in leitender Verbindung steht. Wird das proximale Ende des Katheter in den Spannadapter eingeführt, so legt sich das frei aus dem proximalen Ende des Katheters herausragende Ende des Drahtes aussen an den Katheter an und kommt zwangsläufig in leitenden Kontakt mit der Kontaktbuchse. Um den Draht für die Elektrostimulation zu kontaktieren, sind somit keine anderen Maßnahmen und Handgriffe erforderlich, als sie zum Anbringen eines herkömmlichen Spannadapter notwendig sind. Das Anschließen des Spannadapters an den Stimulator ist einfach und zuverlässig dadurch möglich, dass der elektrische Anschluß zum Beispiel als Steckverbinder ausgebildet ist.

In einer vorteilhaften Ausführung ist die Innenbohrung der Kontaktbuchse konisch ausgebildet, so dass sie sich in Einführungsrichtung des Katheters verengt. Durch diese konische Verjüngung der Kontaktbuchse wird der aussen an dem Katheter anliegende Draht beim Einführen des Katheters verstärkt gegen die Innenwandung der Kontaktbuchse gedrückt, so dass sich ein besonders hoher Kontaktdruck und eine besonders zuverlässige Kontaktierung ergibt.

Im folgenden wird die Erfindung anhand von in der Zeichnung dargestellten Ausführungsbeispielen näher erläutert.

Es zeigen:
- Figur 1: im axialen Halbschnitt eine erste Ausführung des Spannadapters,
- Figur 2: in einer entsprechenden Darstellung eine zweite Ausführung des Spannadapters und
- Figur 3: in einer entsprechenden Darstellung eine dritte Ausführung des Spannadapters.

In allen drei Ausführungsbeispielen weist der Spannadapter einen Spannkörper 10 aus einem isolierenden Kunststoff auf. Der Spannkörper 10 ist in axialer Richtung von einer Bohrung 12 durchsetzt. Am proximalen Ende des Spannkörpers 10 geht die Bohrung 12 koaxial in einen Spritzenansatz 14 über, der zum Beispiel als Luer-Lock-Ansatz ausgebildet sein kann.

Die Bohrung 12 erweitert sich in distaler Richtung mit einer radialen Innenschulter 16. Von der Innenschulter 16 ausgehend erweitert sich die Bohrung 12 in distaler Richtung leicht konisch. Am distalen Ende weist der Spannkörper 10 ein Aussengewinde auf, auf welches ein Spanndeckel 18 nach Art einer Überwurfmutter mit einem Innengewinde aufschraubbar ist. Der Spanndeckel 18 greift mit einer axial vorspringenden Nabe 20 koaxial in die Bohrung 12 ein. Der Spanndeckel 18 und seine Nabe 20 werden koaxial von einer Öffnung 22 durchsetzt.

In die Bohrung 12 ist eine Kontaktbuchse 24 eingesetzt, die die Form eines Hohlzylinders hat und aus einem elektrisch-leitenden Material, insbesondere aus einem Metall besteht. Die Kontaktbuchse 24 liegt axial an der Innenschulter 16 an. Der Aussendurchmesser der Kontaktbuchse 24 entspricht dem Innendurchmesser der erweiterten Bohrung 12, während die Innenbohrung der Kontaktbuchse 24 im Durchmesser mit dem kleineren Durchmesser der Bohrung 12 proximal von der Innenschulter 16 übereinstimmt. Die Innenbohrung der Kontaktbuchse 24 verjüngt sich in proximaler Richtung leicht konisch.

An das distale Ende der Kontaktbuchse 24 schließt sich ein Spannteil 26 an, welches ebenfalls die Form eines Hohlzylinders hat und aus einem elastischen Material, vorzugsweise aus einem elastischen Kunststoff besteht. Der Innendurchmesser und der Aussendurchmesser des Spannteiles 26 stimmen-mit dem-Innendurchmesser und dem Aussendurchmesser der Kontaktbuchse 24 überein, so dass das Spannteil 26 und die Kontaktbuchse 24 im Innendurchmesser und im Aussendurchmesser stufenlos aneinander anschließen. Das Spannteil 26 kann sich entsprechend der Bohrung12 geringfügig in distaler Richtung konisch erweitern.

Beim Aufschrauben des Spanndeckels 18 kommt dessen Nabe 20 axial an der distalen Stirnfläche des Spannteils 26 zur Anlage. Zwischen die Nabe 20 und das Spannteil 26 ist dabei eine Unterlagscheibe 28, vorzugsweise aus Metall, eingesetzt, so dass sich die Nabe 20 beim Aufschrauben des Spanndeckels 18 drehen kann ohne das Spannteil 26 mitzunehmen.

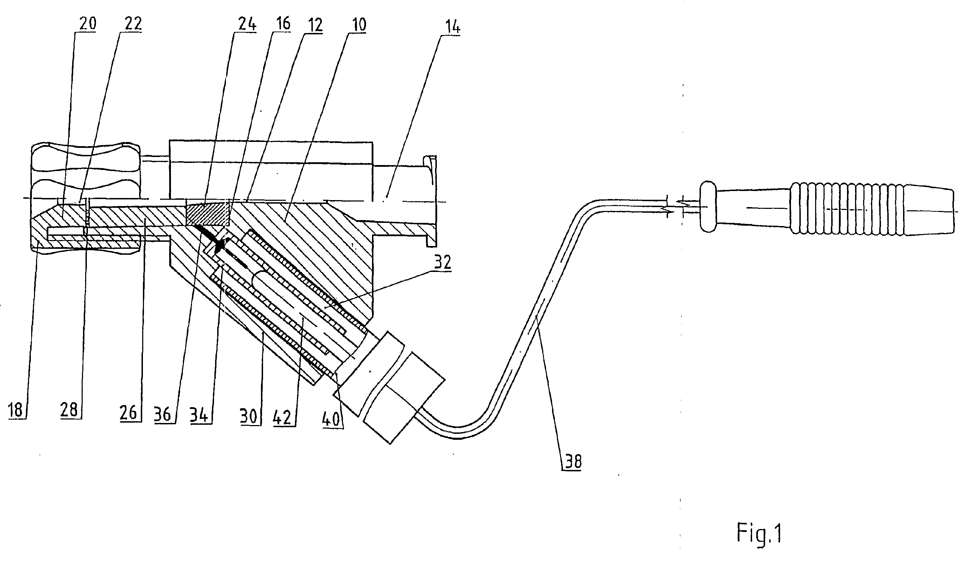

In dem bisher beschriebenen Aufbau stimmen die Ausführungsbeispiele der Figuren 1 bis 3 überein. Auch in Bezug auf das Einführen und Spannen des nicht dargestellten Katheder stimmen diese Ausführungsbeispiele überein.

Zum Einsetzen des Katheters wird der Spanndeckel 18 soweit in distaler Richtung zurückgeschraubt, dass seine Nabe 20 keinen axialen Druck auf das Spannteil 26 ausübt. Der Katheter wird von der distalen Seite durch die Öffnung 22 des Spanndeckels 18 und durch das Spannteil 26 eingeführt bis sein proximales Ende in die Kontaktbuchse 24 gelangt. Der aus dem proximalen Ende des Katheters herausragende Draht für die Elektrostimulation wird dabei radial nach aussen über den Rand des Katheters gebogen, so dass er sich beim Einschieben des Katheters in den Spannadapter um die proximale Randkante des Katheters biegt und sich aussen an den Umfang des Katheters anlegt. Die Innendurchmesser der Öffnung 22, des Spannteils 26 und der Kontaktbuchse 24 sind so auf den Aussendurchmesser des Katheters abgestimmt,dass dieser Katheter leicht, aber mit geringem radialem Spiel eingeführt werden kann. Die Innenbohrung der Kontaktbuchse 24 verjüngt sich dabei jedoch in proximaler Richtung auf einen Innendurchmesser, der gleich oder geringfügig kleiner ist als der Aussendurchmesser des Katheters. Beim Eindringen des proximalen Katheterendes in die Kontaktbuchse 24 wird dadurch der aussen am Umfang des Katheters anliegende Draht durch die Keilwirkung der konischen Verjüngung gegen die Kontaktbuchse 24 gedrückt und kommt in guten elektrisch-leitenden Kontakt mit der Kontaktbuchse 24. Nach dem Einführen des Katheters wird der Spanndeckel 18 in proximaler Richtung aufgeschraubt, so dass seine Nabe 20 in die Bohrung 12 eindringt. Die Nabe 20 drückt dadurch über die Unterlagscheibe 28 axial gegen das Spannteil 26. Da das Spannteil 26 über die Kontaktbuchse 24 an der Innenschulter 16 axial abgestützt ist, führt dies zu einer axialen Stauchung des elastischen Spannteiles 26. Diese Stauchung bewirkt, dass das Spannteil 26 sich radial ausbaucht und seine Wandstärke vergrössert. Da das Spannteil 26 dabei nicht radial nach aussen ausweichen kann, bewirkt dieses Stauchen eine Verringerung des Innendurchmessers des Spannteiles 26, wodurch sich dieses Spannteil 26 unter Druck an den Aussenumfang des Katheters dicht anlegt. Dadurch wird der Katheter in dem Spannadapter gespannt und axial festgehalten. Über eine an dem Spritzenansatz 14 angesetzte Spritze kann nun eine Flüssigkeit, zum Beispiel ein Anästhetikum, durch die Bohrung 12 in den Katheter eingeleitet werden.

Soll der Spannadapter wieder von dem Kathederende abgenommen werden, so ist es nur erforderlich, den Spanndeckel 18 soweit zurückzuschrauben, bis das Spannteil 26 axial entlastet ist, seine ursprüngiche Form wieder annimmt und den Katheter freigibt.

Für die Elektrostimulation muss der in dem Katheder verlaufende Draht in elektrisch leitende Verbindung mit einem Stimulator gebracht werden. Hierzu weist die Kontaktbuchse 24, mit weicher der Draht in leitender Berühung steht, einen durch das Kunststoffgehäuse des Spannkörpers 10 nach aussen geführten elektrischen Anschluss auf. Die Figuren 1 bis 3 zeigen unterschiedliche Ausgestaltungen dieses Anschlusses.

In dem Ausführungsbeispiel der Figur 1 weist der Spannkörper 10 eine seitliche Verbreiterung 30 auf, deren Aussenkontur mit der Mittelachse der Bohrung 12 einen sich in proximaler Richtung öffenden Winkel von etwa 45° einschließt. In dieser Verbreiterung 30 ist eine sacklochförmige Steckeraufnahme 32 ausgebildet, deren Achse ebenfalls unter diesem Winkel von 45° läuft. In der Steckeraufnahme 32 ist koaxial eine leitende Steckerbuchse 34 angeordnet, die am Grund der Stekkeraufnahme 32 über einen leitenden Stift 36 mit der Kontaktbuchse 24 in Verbindung steht. Zum Anschließen des nicht dargestellten Stimulators dient ein mit dem Stimulator verbundenes Kabel 38, welches mit einem Stecker 40 versehen ist, der in die Steckeraufnahme 32 einsteckbar ist, wobei ein Steckerstift 42 des Steckers 40 die Steckerbuchse 34 kontaktiert.

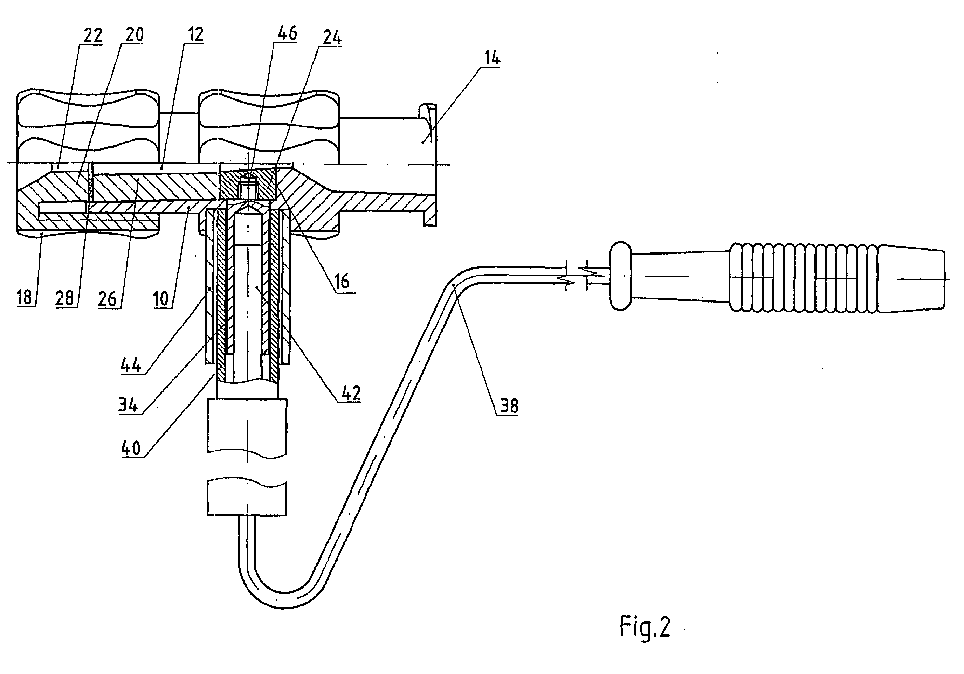

Bei dem Ausführungsbeispiel der Figur 2 ist der elektrische Anschluß für den Stimulator rechtwinklig zur Achse des Spannadapters angeordnet. In den Spannkörper 10 ist ein radial abstehendes Kunststoffrohr 44 eingesetzt, welches die Stekkeraufnahme bildet. In dem Kunststoffrohr 44 ist koaxial die Steckerbuchse 34 angeordnet, die mit einem Einschraubstift 46 in die Kontaktbuchse 24 eingeschraubt ist. Auch hier wird ein Anschlußkabel 38 mit einem Stecker 40 in die Steckeraufnahme und die Steckerbuchse 34 eingesteckt.

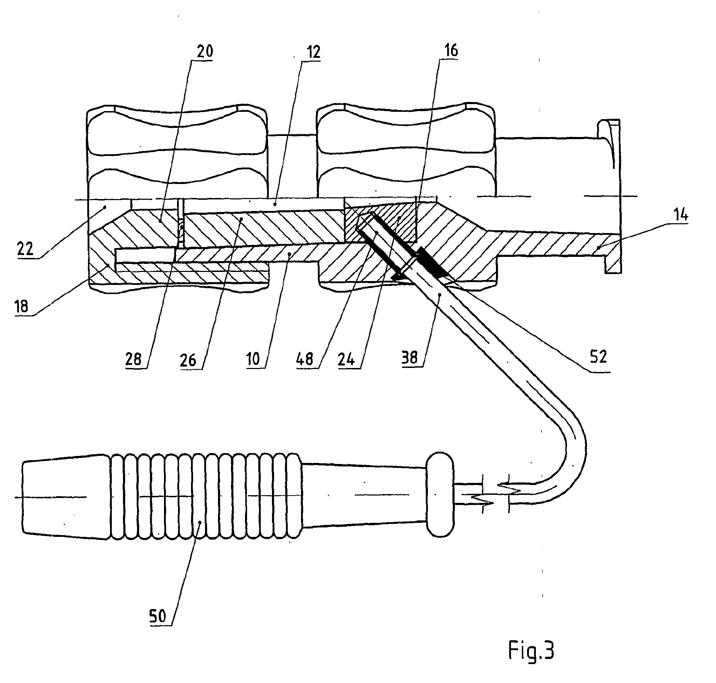

In dem Ausführungsbeispiel der Figur 3 ist ein Kontaktstift 48 unter einem Winkel von 45° zur Achse der Bohrung 12 in die Kontaktbuchse 24 eingesetzt. An den Kontaktstift 48 ist ein Kabel 38 angeschlossen, an dessen anderem Ende ein Steckverbinder 50 für den Anschluß des Stimulators vorgesehen ist. Der Kontaktstift 48 mit dem angelöteten Kabel 38 ist in den Spannkörper 10 eingegossen oder mittels eines Klebers 52 eingeklebt.

### Bezugszeichenliste

- 10: Spannkörper
- 12: Bohrung
- 14: Spritzenansatz
- 16: Innenschalter
- 18: Spanndeckel
- 20: Nabe
- 22: Öffnung
- 24: Kontaktbuchse
- 26: Spannteil
- 28: Unterlagscheibe
- 30: Verbreiterung
- 32: Steckeraufnahme
- 34: Steckerbuchse
- 36: Stift
- 38: Kabel
- 40: Stecker
- 42: Steckerstift
- 44: Kunststoffrohr
- 46: Einschraubstift
- 48: Kontaktstift
- 50: Steckverbinder
- 52: Kleber

## Patentansprüche

1. Spannadapter für einen Katheter mit einem Spannkörper (10) aus einem Isolierstoff, mit einer den Spannkörper (10) axial durchsetzenden Bohrung (12), mit einem proximal an dem Spannkörper (10) angeordneten Spritzenansatz (14), in den sich die Bohrung (12) fortsetzt, mit einem hohlzylindrischen elastischen Spannteil (26), welches von distal in die Bohrung (12) einsetzbar ist und in proximaler Richtung axial abgestützt ist, und mit einem Spanndeckel (18), der distal auf den Spannkörper (10) aufsetzbar ist und an der distalen Stimfläche des Spannteils (26) angreift, wobei der Katheter durch eine Öffnung (22) des Spanndeckels (18) hindurch axial in das Spannteil (26) einführbar ist und durch das mittels Spanndeckels (18) axial gestauchten Spannteils (26) spannbar ist, **dadurch ge-kennzeichnet, dass** zum Kontaktieren eines in dem Katheter angeordneten Drahtes für die Elektrostimulation in dem Spannkörper (10) eine elektrisch-leitende Kontaktbuchse (24) proximal an das Spannteil (26) anschließend angeordnet ist, deren Innenbohrung koaxial an die Innenbohrung des Spannteils (26) anschließt, und dass die Kontaktbuchse (24) einen durch den Spannkörper (10) nach aussen geführten elektrischen Anschluß aufweist.

2. Spannadapter nach Anspruch 1,**dadurch gekennzeichnet, dass** die Kontaktbuchse (24) axial zwischen eine Innenschulter (16) der Bohrung (12) und das Spannteil (26) eingesetzt ist und das Spannteil (26) axial abstützt.

3. Spannadapter nach Anspruch 1 oder 2, **dadurch gekennzeichnet, dass** die Kontaktbuchse (24) eine sich in proximaler Richtung konisch verjüngende Bohrung aufweist.

4. Spannadapter nach einem der Ansprüche 1 bis 3, **dadurch gekennzeichnet, dass** der elektrische Anschluß einen in dem Spannkörper (10,30,44) aufgenommen und mit der Kontaktbuchse (24) elektrisch verbundenen Steckverbinder (34) aufweist.

5. Spannadapter nach einem der Ansprüche 1 bis 3, **dadurch gekenn-zeichnet, dass** der elektrische Anschluß ein mit der Kontaktbuchse (24) leitend verbundenes Kabel (38) aufweist, an dessen anderem Ende ein Steckverbinder (50) angebracht ist.

## Claims

1. A clamp adapter for a catheter, having a clamping body (10) made from an insulating material, having a bore (12) passing axially through the clamping body (10), having a syringe fitting (14) disposed proximally to the clamping body (10), in which fitting the bore (12) continues, having a hollow cylindrical, elastic clamping part (26), which can be inserted into the bore (21) from the distal side and is axially supported in the proximal direction, and having a clamping lid (18), which can be placed distally onto the clamping body (10) and abuts the distal end face of the clamping part (26), it being possible to insert the catheter through an opening (22) in the clamping lid (18) axially into the clamping part (26) and to clamp it by a clamping part (26) that is axially upset by means of the clamping lid (18),
**characterised in that**, to provide a contact for a wire disposed in the catheter for electrical stimulation in the clamping body (10), an electrically conductive contact bushing (24) is disposed proximally adjacent to the clamping part (26), the internal bore of which is coaxially adjacent to the inner bore of the clamping part (26),
**and in that** the contact bushing (24) comprises an electrical connection passed outwardly through the clamping body (10).

2. A clamp adapter according to Claim 1,
**characterised in that** the contact bushing (24) is inserted axially between an internal shoulder (16) of the bore (12) and the clamping part (26) and axially supports the clamping part (26).

3. A clamp adapter according to Claim 1 or 2,
**characterised in that** the contact bushing (24) comprises a bore tapering conically in the proximal direction.

4. A clamp adapter according to one of Claims 1 to 3,
**characterised in that** the electrical connection comprises a plug connector (34) that is housed in the clamping body (10, 30, 44) and is electrically connected to the contact bushing (24).

5. A clamp adapter according to one of Claims 1 to 3,
**characterised in that** the electrical connection comprises a cable (38) connected conductively to the contact bushing (24), at the other end of which a plug connector (50) is mounted.

## Revendications

1. Adaptateur de serrage pour un cathéter comprenant un corps de serrage (10) en matière isolante, un alésage (12) traversant axialement le corps de serrage (10), une embase de seringue (14) du côté proximal sur le corps de serrage (10) dans lequel l'alésage (12) se prolonge, une pièce de serrage élastique en forme de cylindre creux (26), qui peut être mis du côté distal dans l'alésage (12) et qui est appuyé axialement dans la direction proximale, et un couvercle de serrage (18) qui peut être mis du côté distal sur le corps de serrage (10) et qui s'applique sur la surface frontale distale de la pièce de serrage (26), le cathéter pouvant être introduit axialement dans la pièce de serrage (26) par une ouverture (22) du couvercle de serrage (18) et être mis sous contrainte au moyen d'une pièce de serrage (26) comprimée axialement au moyen du couvercle de serrage (18),
**caractérisé en ce que**
pour la mise en contact d'un fil disposé dans le cathéter en vue de l'électrostimulation dans le corps de serrage (10), une douille de contact électriquement conductrice (24) se raccorde du côté proximal à la pièce de serrage (26), et la douille de contact (24) présente un raccordement électrique vers l'extérieur à travers le corps de serrage (10).

2. Adaptateur de serrage selon la revendication 1,
**caractérisé en ce que**
la douille de contact (24) est introduite axialement entre un épaulement intérieur (16) de l'alésage (12) et la pièce de serrage (26), et elle soutient la pièce de serrage (26) dans le sens de son axe.

3. Adaptateur de serrage selon la revendication 1 ou 2,
**caractérisé en ce que**
la douille de contact (24) présente un alésage qui se rétrécit sous une forme conique dans la direction proximale.

4. Adaptateur de serrage selon l'une quelconque des revendications 1 à 3,
**caractérisé en ce que**
le raccordement électrique présente un connecteur d'enfichage (34) pris dans le corps de serrage (10, 30, 44) et électriquement connecté à la douille de contact (24).

5. Adaptateur de serrage selon l'une quelconque des revendications 1 à 3,
**caractérisé en ce que**
le raccordement électrique présente un câble (38) relié de façon conductrice à la douille de contact (24), et dont l'autre extrémité comporte un connecteur enfichable.
